# EUROPEAN PATENT SPECIFICATION

(11) **EP 2 068 711 B1**
(45) Date of publication and mention of the grant of the patent: **15.01.2014**
(21) Application number: 06806100.1
(22) Date of filing: 06.10.2006
(51) Int. Cl.: A61B 5/113

(54) **RESPIRATION SENSOR**
ATEMSENSOR
CAPTEUR DE RESPIRATION

(43) Date of publication of application: 17.06.2009
(73) Proprietor: Elekta AB (publ), 103 93 Stockholm (SE)
(72) Inventor: WUNDERINK, Wouter, 3076 RE Rotterdam (NL)
(74) Representative: Downing, Michael Philip
(86) International application number: PCT/EP2006/009700
(87) International publication number: WO 2008/040379

(56) References cited:
- WO-A-2005/044090
- DE-A1-102004 045 495
- US-A- 5 681 326

## Description

### FIELD OF THE INVENTION

The present invention relates to respiration monitoring.

### BACKGROUND ART

Respiration monitoring is useful during a range of treatments, including radiotherapy. As radiotherapy involves directing harmful beams of radiation toward a tumour, it is important to minimise the dose that is applied to healthy tissue. Collimators are used to shape the beam appropriately, and the beam direction is varied over time, and these can assist in delivering the maximum dose to a tumour area and a minimum dose to healthy tissue.

These measures require the location of the tumour to be known. Generally, this is established through prior investigation, but tumours near to the lungs present difficulty in that they move with the surrounding tissue as the respiration cycle progresses. Generally, the tumour will only be at the expected location at like points in the respiration cycle.

Respiration monitoring is also important in imaging applications. Computed Tomography (CT) scanners can produce very good quality three dimensional representations from a series of two-dimensional images, but assume that the patient has not moved or changed between the successive images. Respiration obviously breaches this assumption and therefore causes artefacts or degradation in the 3D representation.

To date, this has been dealt with either by prompting the respiration cycle with indicators that direct the patient when to breathe, or by detecting the cycle. Our earlier application W02004/066211 described an algorithm that could detect the respiration phase from cone beam CT images and select the appropriate images for processing. Most prior art used to detect the respiration cycle relies on the detecting the motion that results from respiration.

WO2005/044090 discloses a wearable platform embodied in a belt. The wearable platform includes respiration sensors.

### SUMMARY OF THE INVENTION

This invention seeks to simultaneously minimise or even eliminate the respiratory motion while detecting the respiratory cycle.

The present invention therefore provides a respiration sensor as set out in claim 1.

The patient support will usually support the patient in a generally horizontal state. Thus, the frame can extend around the patient in the form of a support beneath the patient and a first point of contact above the patient.

The frame is preferably radiolucent thereby to avoid interfering with the radiological investigation taking place. The frame can further comprise a radiolucent fixing means arranged inside the frame and on which the patient's body is intended to rest, said fixing means comprising a flexible casing which contains a yieldable substance, the fixing means being adapted to permit the patient to be partly-sunk into the fixing means and oriented in a desired position at a first stage, and the yieldable substance of the fixing means being transformable Into a non-yielding state to fix the patient in the desired position at a second stage. Such a frame is shown in US 5,681,326.

The frame can include an arch extending over an area adapted to receive a patient. An arch is advantageous in that it is able to connect to the frame on both sides of the patient and thereby offer a more rigid substrate-from which to measure forces exerted by the patient.

The first point of contact can include a plate which abuts the patient. A plate will offer a greater surface area and therefore inflict less discomfort on the patient. The plate can be attached to the frame via an adjustable means, for example, to cater for different sizes of patient. A screw thread offers suitable adjustability. The sensor is ideally located between the adjustable means and the plate. The adjustable means can be attached to the frame at the arch, thereby enabling it to be placed directly over the patient's abdomen. This will ensure that, in use, it is placed purely in compression rather than being subjected to bending moments. Again, this assists in ensuring the rigidity of the overall structure.

### BRIEF DESCRIPTION OF THE DRAWINGS

An embodiment of the present invention will now be described by way of example, with reference to the accompanying figures in which;
Figure 1 shows a patient prior to treatment in a body frame;
Figure 2 shows a vertical section through the frame of figure 1;
Figure 3 shows a sagital CT-slice of the patient of figure 1;
Figure 4 shows a sensor according to the present invention;
Figure 5a shows a schematic illustration from above of such a sensor;
Figure 5b shows a schematic exploded illustration of such a sensor;
Figure 6 shows the response of such a sensor to a force exerted thereon;
Figure 7 shows a fitting location for the sensor of figure 4;
Figure 8 shows an alternative to figure 7;
Figure 9 shows the data processing apparatus;
Figure 10 shows the motion in a patient detected during respiration; and
Figure 11 shows the detected motion together with the force sensed according to the present invention.

### DETAILED DESCRIPTION OF THE EMBODIMENTS

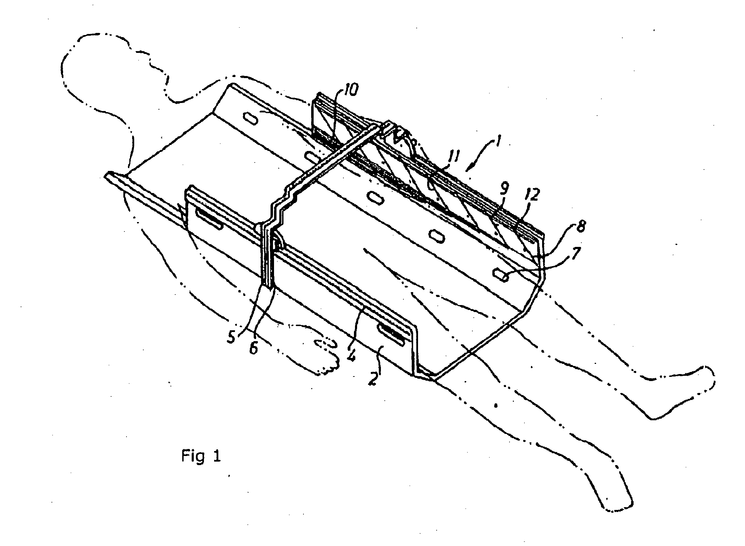

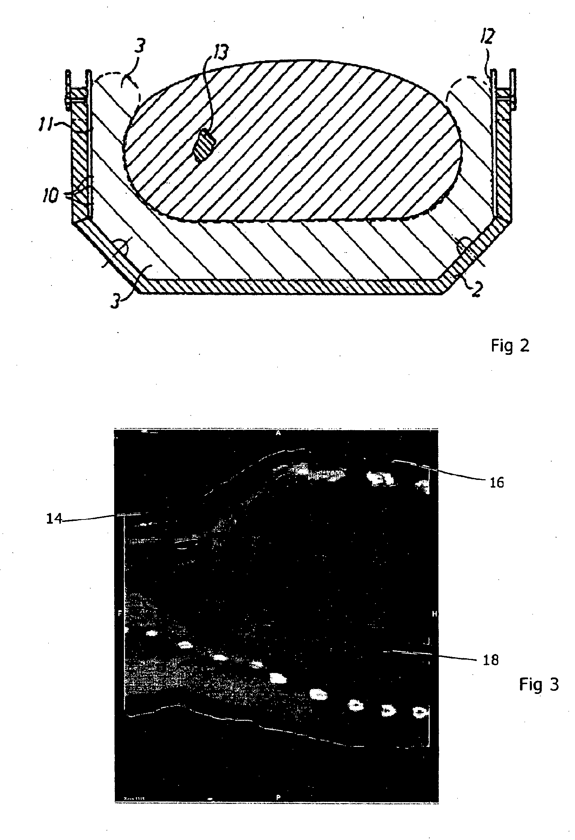

Figure 1 shows a stereotactical body frame 1 as can be used in the present invention. This comprises an elongate, non-yielding frame 2 which is made of a material that does not cause any artefacts (disturbances) in the images, i.e., a material which is translucent to X-rays and other radioactive radiation. A fixing means 3 is adapted to be inserted in the frame 2 which is open at the top and at the ends, said fixing means comprising a flexible casing that is impermeable to fluid and hermetically encloses a yielding substance, not shown. On this fixing means, the patient is caused to take the desired position for diagnostics or treatment, the patient partially sinking into the yieldable fixing means, and a large contact surface against the patient is obtained, see figure 2. Subsequently, the fixing means 3 is given a non-yielding state so as to fix the patient in the desired position. This can be achieved by the casing of the fixing means holding a plurality of small bodies, for example so-called Frigolit beads or the like, which are fixed in their respective positions when a negative pressure is produced in the impermeable casing. It is also possible to fill the casing with a fluid which can be caused to solidify so as to keep its enforced shape. Thus, the casing can be filled with prepolymerised polyurethane foam which, when the patient has been oriented in the desired position, is caused to finally polymerise and thus form a non-yielding, fixing compound. By means of a first indicating device 4 fixedly arranged on the frame 2 in the longitudinal direction thereof, and a second indicating device 5 arranged on an arch 6 which is displaceable on the frame in the longitudinal direction thereof, the patient can be oriented in the fixing means for treatment of an area which has previously been located by means of computerised tomography, X-rays etc.

The frame 2 is preferably formed with fixing holes (not shown) into which the fixing means can penetrate at its first, yielding stage, and/or fixing lugs 7 penetrating into the fixing means so as to fix this at its second, non-yielding stage.

Finally, the frame 2 comprises one or more orienting means 8 along each longitudinal side of the frame. The orienting means 8 which preferably is releasably attached to the inside of the frame, consists of a radiolucent support with three arrays of lines preventing radiation, which are made of, e.g., wire, strips or thin sections of copper for diagnostic X-ray, angiography, DSA and CT examinations or gas-, liquid- or substance-filled tubes for diagnostic PET and MRI examinations. The first array 10 comprises a number of lines of different lengths extending in parallel from one open end of the frame, said lines preferably being parallel also with the bottom of the frame, as shown in figure 1. The second array 11 comprises parallel lines extending at an angle to the first array 10 and extending from the first array along each longitudinal side of the frame. The second array 11 is terminated at a third array 12 adjacent the upper edge of the longitudinal side. The third array 12 comprises one or more unbroken lines which preferably are parallel with the lines of the first array. See also figure 2 which schematically illustrates a vertical section of the orienting means 8. In this Figure, the frame 2 is indicated by full lines and the upper surface of the fixing means 3 by dashed lines.

The frame 2 is supported by a bed unit which allows the patient to be moved while fixed in the frame.

The diagnostic equipment produces an image section, for example an MRI or CT sectional image, and the orientation of this section is indicated by the orienting means 8. By counting the number of lines in the first array 10, a base value is obtained. The distance between the uppermost unbroken line in the first array and the point in which the image section cuts one of the inclined lines in the second array is measured and yields a supplementary value, see figure 2. Since the distance between the two upper horizontal marks 11 and 12 is known, this distance can be used as a reference for calculating the vertical coordinate in the image sectional plane. Similarly, the horizontal coordinate in the image plane can be calculated by the distance between 11 and 12 on the two sides of the frame being known. By means of these values, the orientation of the image section can be determined with great accuracy. It should be noted that the image section need not be oriented at right angles to the longitudinal axis of the frame 2, but may be oriented at an optional angle, as shown in figure 2 in which each point of intersection between the image section and the associated line in the second array 11 is positioned on different levels. This Figure schematically illustrates a sectional image of the patient in which the target area or treatment area 13 appears. In diagnostic use, the orienting accuracy of the stereotactical system has been about 1 mm, and in repeated treatment using the stereotactical instrument the orienting accuracy has been about 3 mm in the transverse plane and about 6 mm in the longitudinal plane. These values were obtained for target areas close to the diaphragm and were to a certain extent affected by the patient's breathing. In order to reduce the effect of the breathing movements, an abdominal compression plate (14) has been used on such occasions.

The stereotactical coordinates are adjusted on the scales 4 and 5 in the treatment room by means of prior-art wall-mounted lasers, mounted-and set in conventional manner, which define the system of coordinates for the room/treatment unit. Guided by the values which are obtained in the locating of the target point, i.e. the tissue area To be treated, and which are transferred to the stereotactical instrument, the coordinates are obtained which are necessary to be able to set a radiation source, starting from the frame, such that the beam therefrom is directed to the treatment site or target point.

Figure 3 shows the type of image that is available through the use of such a frame. Even with an abdominal compression plate 14 in place (visible in the image), there are visible artefacts 16, 18 in the image that result from the patient's respiration cycle. Thus, although the body frame offers great advantages in supporting the patient, especially during second or subsequent visits, there is clearly scope for improvement.

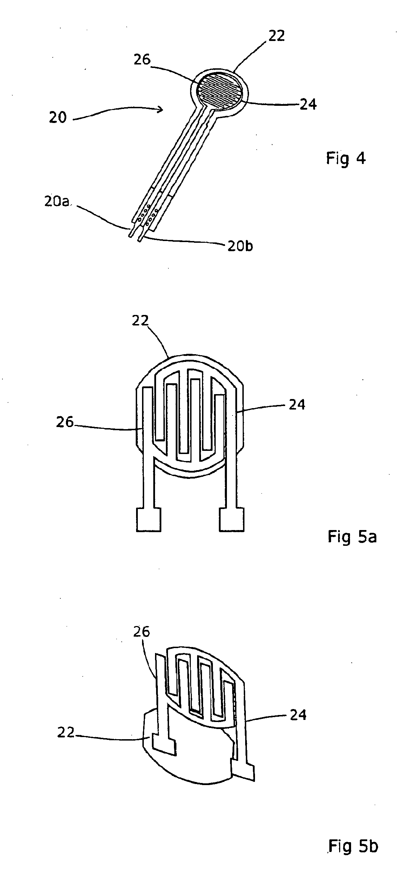

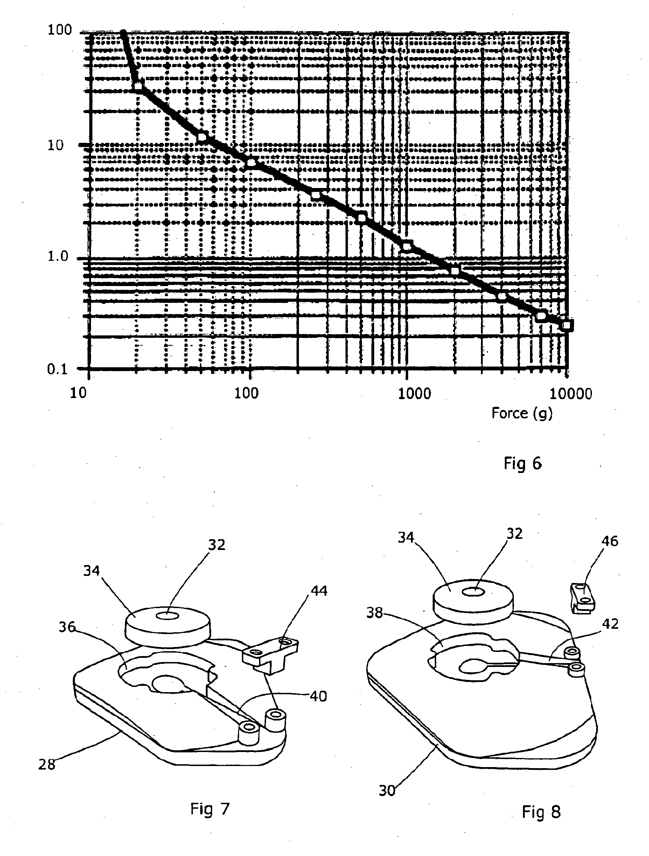

Figure 4 shows a force sensor 20. As their name implies, force sensing resistors use the electrical property of resistance to measure the force (or pressure) applied to them. A force sensing resistor is made up of two parts, a resistive material 22 carried on a film and a set of interdigitating contacts 24, 26 also carried on a film. Each respective contact 24, 26 is connected to one of the two lead-out wires 20a, 20b. This is apparent in figure 4, but figures 5a and 5b shows a simplified pattern in more detail. The resistive material serves to make an electrical path between the two sets of conductors on the other film. When a force is applied to this sensor, a better connection is made between the contacts, and hence the conductivity is increased. Over a wide range of forces, it turns out that the conductivity is approximately a linear function of force as shown in figure 6, the resistance of a typical sensor as a function of force.

Figures 7 and 8 show abdominal compression plates 28, 30 suitable for use in the present invention. Generally, these are held in place via a threaded shaft (not shown) that passes through a threaded aperture in the arch 5 or a like structure in the body frame, and which seats in a blind recess on 32 on a washer 34. That washer 34 fits in a corresponding recess 36, 38 in the compression plates 28, 30. Thus, once the patient is in position in the body frame, the arch 5 (or other structure) is erected, the shaft is threaded into its hole in the arch 5 (or other structure), and the compression plate 28 or 30 with washer 34 is placed in position on the patient. The shaft is then rotated to drive it downwards into the recess 32, pressing the plate 28 or 30 into the patient's abdomen. This assists in holding the patient in place and in (to an extent) limiting respiratory movement of the patient's organs. Washer 32 assists in limiting the transfer of rotational forces from the shaft to the compression plate 28, 30.

Figures 7 and 8 show a channel 40, 42 in each of the compression plates 28, 30 leading from the recess 36, 38 to an edge of the plate 28, 30. At the edge of the plate 28, 30, the channels 40, 42 end with a cable grip 44, 46. In the plate 28 of figure 7, the channel 40 leads away from the recess 36 diagonally with respect to the patient whereas in the plate 30 of figure 8, the channel 42 leads away from the recess 38 longitudinally with respect to the patient. The choice is largely a matter of convenience and comfort for the patient.

These channels 40, 42 allow a force sensing resistor 20 to be placed in the compression plate 28, 30. The active area of the resistor 20 can then be sandwiched between the plate 28, 30 and the washer 32, within the recess 36, 38. To obtain reliable operation, it assists to place a flexible material (preferably one that shows few hysteretic effects after a release of pressure) in between the washer 32 and the resistor 20, in the channel 40, 42 of plate 28, 30. We prefer to fill the channel 40, 42 with silicone and use a 2mm soft rubber layer on top, which partially makes contact with the plate 28,30, and partially with the resistor 20. In this way, part of the pressure exerted by washer 32 can be directed straight to the plate, thereby preventing the sensor from saturating at the slightest force on the washer.. The lead-out connections 20a, 20b attached to the resistor 20 can be placed in the channel 40, 42 and are secured by the cable grip 44, 46.

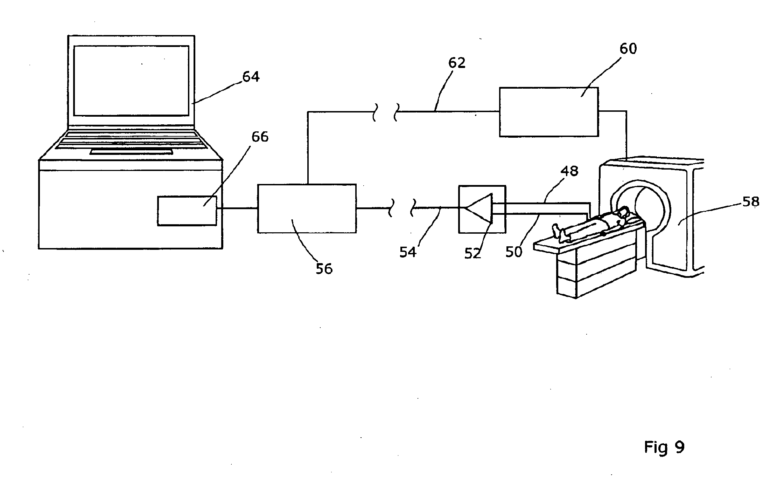

Figure 9 shows the processing of the information received from the force sensing resistor 20. The lead-out wires 20a, 20b are connected to conductors 48.50 within a cable leading to a driver/amplifier unit 52 which supplies a current to the resistor 20, detects the voltage drop, and amplifies this to a useful signal level. That signal is passed via a cable 54 to a connection unit 56 which also receives imaging data from the CT scanner 58 via a sensor port 60 and connecting cable 62.

The imaging data and the respiratory signal data from the resistor 20 are both then fed to a computing means 64, the respiratory signal data passing via an analogue to digital converter 66. The respiratory signal data can then be used to select samples from the imaging data that correspond to like-phase points in the respiratory cycle. These selected images can be used to construct a three dimensional tomography via known computing methods.

In another example in which the Siemens Sensation Open CT-scanner is used, employing the Anzai sensor-port system, the signal entering the connection unit 56 (via 54) is duplicated within unit 56. The signal is directed via 62 to sensor port 60 and can also be received by 66. The sensor port provides the respiration signal to the CT-scanner computer, where it is stored together with the imaging data. In other words, in this example unit 56 is a simple rewiring device without any active components, and could simply be replaced by two separate cables. For CT applications, the computer 64 is only used for monitoring the signal, to adjust the right offset and gain on the amplifier unit 52. In a kV fluoroscopy application, the computer 64 is used to store the respiration signal, together with kV-images provided to this computer.

Alternatively, the respiratory signal data can be analysed in real time and used to gate the CT scanner 58, thereby creating images that are phase-coordinated. This will have the advantage that the patient will be exposed to less radiation, but the disadvantage that only one point in the phase can be imaged. Offline selection of the images after exposure allows substantially any required phase to be chosen.

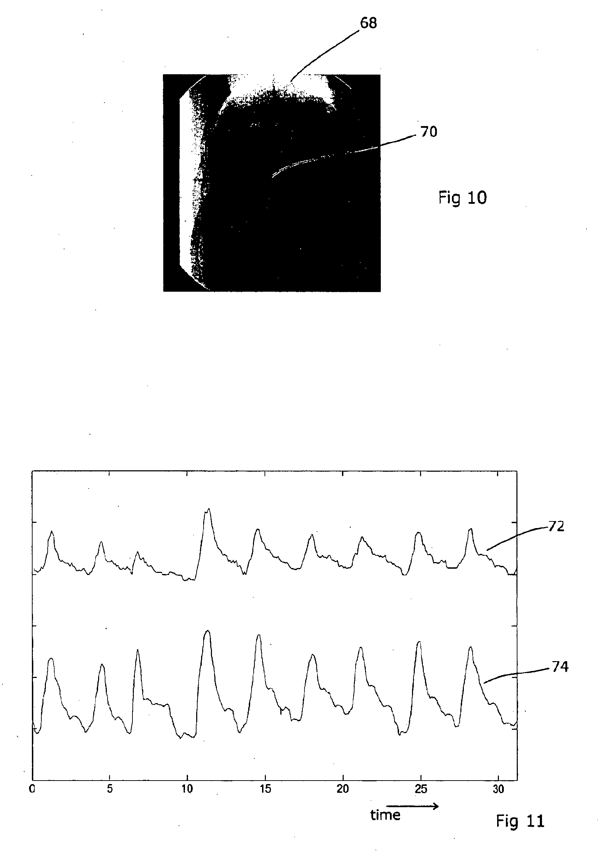

Figure 10 shows a digital x-ray fluoroscopic image 68 of gold markers implanted into a patient. Each marker such as that referenced 70 can be seen to leave a line in the time-averaged image showing the movement of that local area if tissue during respiration. Figure 11 shows an upper line 72 that is the instantaneous location of one such marker with time. A lower line 74 is the monitored force reported by the force sensing resistor 20. It can be seen that the two lines show a variation that exhibits a periodic variation that has the same phase; therefore it can be concluded that the resistor 20 accurately reports breathing phase.

It will of course be understood that many variations may be made to the above-described embodiment without departing from the scope of the present invention.

## Claims

1. A respiration sensor, comprising a non-yielding frame (2) that is adapted to extend from a first point of contact on one side of a patient to a patient support permitting a second point of contact on an opposing side of the patient, including a force sensor (20) at the first point of contact.

2. A respiration sensor according to claim 1 in which the frame comprises a connector for fitting the sensor to the patient support.

3. A respiration sensor according to claim 1 in which the patient support supports the patient in a generally horizontal state.

4. A respiration sensor according to any one of the preceding claims in which the frame is radiolucent.

5. A respiration sensor according to any one of the preceding claims in which the frame further comprises a radiolucent fixing means (3) arranged inside the frame and on which the patient's body is intended to rest, said fixing means comprising a flexible casing which contains a yieldable substance, the fixing means being adapted to permit the patient to be partly sunk into the fixing means and oriented in a desired position at a first stage, and the yieldable substance of the fixing means being transformable into a non-yielding state to fix the patient in the desired position at a second stage.

6. A respiration sensor according to any one of the preceding claims in which the frame includes an arch (6) extending over an area adapted to receive a patient.

7. A respiration sensor according to any one of the preceding claims in which the first point of contact includes a plate (28, 30) which abuts the patient.

8. A respiration sensor according to claim 7 in which the plate is attached to the frame via an adjustable means.

9. A respiration sensor according to claim 8 in which the adjustable means is a screw thread.

10. A respiration sensor according to claim 8 and claim 9 in which the sensor is located between the adjustable means and the plate.

11. A respiration sensor according to any one of claims 8 to 10 as dependent on claim 6 in which the adjustable means is attached to the frame at the arch.

## Patentansprüche

1. Atmungssensor, umfassend einen unnachgiebigen Rahmen (2), der daran angepasst ist, stich von einem ersten Berührungspunkt auf einer Seite eines Patienten zu einer Patientenhalterung, die einen zweiten Berührungspunkt auf einer gegenüberliegenden Seite des Patienten gestattet, zu erstrecken, einschließlich eines Kraftsensors (20) am ersten Berührungspunkt.

2. Atmungssensor nach Anspruch 1, wobei der Rahmen Verbinder zum Anbringen des Sensors an der Patientenhalterung umfasst.

3. Atmungssensor nach Anspruch 1, wobei die Patientenhalterung den Patienten in einem generell horizontalen Zustand hält.

4. Atmungssensor nach irgendeinem der vorangehenden Ansprüche, wobei der Rahmen strahlendurchlässig ist.

5. Atmungssensor nach irgendeinem der vorangehenden Ansprüche, wobei der Rahmen ferner ein strahlendurchlässiges Befestigungsmittel (3) umfasst, das innerhalb des Rahmens angeordnet ist und auf welchem der Körper des Patienten aufliegen soll, wobei das Befestigungsmittel ein flexibles Gehäuse umfasst, welches eine nachgiebige Substanz enthält, wobei das Befestigungsmittel daran angepasst ist, den Patienten teilweise in das Befestigungsmittel hinunterlegen und in einer gewünschten Position in einer ersten Stufe ausrichten zu lassen, und wobei die nachgiebige Substanz des Befestigungsmittels in einen unnachgiebigen Zustand umwandelbar ist, um den Patienten in der gewünschten Position in einer zweiten Stufe zu befestigen.

6. Atmungssensor nach irgendeinem der vorangehenden Ansprüche, wobei der Rahmen einen Bogen (6) umfasst, der sich über einen Bereich erstreckt, der an das Aufnehmern eines Patienten ist.

7. Atmungssensor nach irgendeinem der vorangehenden Ansprüche, wobei der erste Berührungspunkt eine Platte (28, 30) umfasst, welche an dem Patienten anliegt.

8. Atmungssensor nach Anspruch 7, wobei die Platte über ein einstellbares Mittel am Rahmen befestigt ist.

9. Atmungssensor nach Anspruch 8, wobei das einstellbare Mittel ein Schraubengewinde ist.

10. Atmungssensor nach Anspruch 8 und Anspruch 9, wobei der Sensor zwischen dem einsteilbaren Mittel und der Platte angeordnet ist.

11. Atmungssensor nach irgendeinem der Anspruche 8 bis 10, soweit abhängig von Anspruch 6, wobei das einstellbare Mittel am rahmen an dem Bogen befestigt ist.

## Revendications

1. Capteur de respiration, comprenant un cadre non élastique (2) adapté pour s'étendre depuis un premier point de contact situé sur un côté dru corps d'un patient jusqu'à un support pour le patient rendant possible un second point de contact situé sur un côté opposé du corps du patient, équipé d'un capteur de force (20) au niveau du premier point de contact.

2. Capteur de respiration selon la revendication 1, dans lequel le cadre comprend un connecteur pour fixer le capteur au support pour le patient.

3. Capteur de respiration selon la revendication 1, dans lequel le support pour le patient supporte le patient dans une position généralement horizontale.

4. Capteur de respiration selon l'une quelconque des revendications précédentes, dans lequel le cadre est transparent au rayonnement.

5. Capteur de respiration selon l'une quelconque des revendications précédentes, dans lequel le cadre comprend en outre un moyen de fixation transparent au rayonnement (3) disposé à l'intérieur du cadre est sur lequel le corps du patient est destiné à venir prendre appui, ledit moyen de fixation comprenant un boîtier flexible qui contient une substance élastique, le moyen de fixation étant adapté pour permettre au patient d'être partiellement enfoncé dans le moyen de fixation et dirigé dans une position souhaitée dans une premier temps, et la substance élastique du moyen de fixation pouvant être transformée dans un état non élastique pour fixer le patient dans la position souhaitée dans un second temps.

6. Capteur de respiration selon l'une quelconque des revendications précédentes, dans lequel le cadre comprend une arcade (6) s'étendant au-dessus d'une zone aménagée pour accueillir un patient.

7. Capteur de respiration selon l'une quelconque des revendications précédentes, dans lequel le premier point de contact comprend une plaque (28, 30) qui vient se placer contre le patient.

8. Capteur de respiration selon la revendication 7, dans lequel la plaque est ahachée au cadre par l'intermédiaire d'un moyen réglable.

9. Capteur de respiration selon la revendication 8, dans lequel le moyen réglable est un filetage de vis.

10. Capteur de respiration selon la revendication 8 et la revendication 9, dans lequel le capteur est situé entre le moyen réglable et la plaque.

11. Capteur de respiration selon l'une quelconque des revendications 8 à 10 en fonction de la revendication 6, dans lequel le moyen réglable est attaché au cadre au niveau de l'arcade.
